# EUROPÄISCHE PATENTANMELDUNG

(11) **EP 0 816 383 A1**
(43) Veröffentlichungstag der Anmeldung: **07.01.1998**
(21) Anmeldenummer: 97109810.8
(22) Anmeldetag: 17.06.1997
(51) Int. Cl.: C08F 2/34, A61L 15/00, C08F 220/06

(54) **Verfahren zur Herstellung von hydrophilen, hochquellfähigen Hydrogelen**

(30) Priorität: 24.06.1996 DE 19625143
(71) Anmelder: Clariant GmbH, 65929 Frankfurt am Main (DE)
(72) Erfinder: Engelhardt, Fritz, Dr., Virginia 23320 (US); Mayer, Manfred, Dr., 65527 Niedernhausen (DE); Nickel, Uwe, Dr., 61532 Bad Homburg (DE)

(57) **Zusammenfassung**

Die vorliegende Erfindung betrifft ein Verfahren zur Herstellung von hydrophilen, hochquellfähigen Hydrogelen durch (Co)polymerisation von hydrophilen Monomeren im Wirbelbettapparat.

## Beschreibung

Die vorliegende Erfindung betrifft ein Verfahren zur Herstellung von hydrophilen, hochquellfähigen Hydrogelen durch (Co)polymerisation von hydrophilen Monomeren im Wirbelbettapparat.

Hydrophile, hochquellfähige Hydrogele sind beispielsweise Polymere aus (co)polymerisierten hydrophilen Monomeren. Solche Hydrogele werden als wäßrige Lösungen absorbierende Produkte zur Herstellung von Windeln, Tampons, Damenbinden und anderen Hygieneartikeln, aber auch als wasserzurückhaltende Mittel in Landwirtschaft und Gartenbau verwendet.

Zur Herstellung solcher Hydrogele sind Verfahren bekannt, bei denen Monomere, wie beispielsweise Acrylsäure und Methacrylsäure in wässriger Lösung unter Zugabe von Katalysatoren, Initiatoren und Vernetzer sauer oder bis zu einem gewissen Prozentsatz neutralisiert, polymerisiert werden. Dabei wird die Reaktionslösung mit Wasser soweit verdünnt, daß sich nach der Reaktion ein Gel bildet. Die Verdünnung mit Wasser wird einerseits zur Begrenzung der maximalen Temperatur, die durch Erwärmung infolge der Polymerisationswärme auftritt, vorgenommen, andererseits setzt die Verarbeitbarkeit des Gels zu Granulaten dem Feststoffgehalt Grenzen. Die Reaktion wird in der Regel unter Erwärmung entweder im ruhenden Zustand (Topfreaktor oder Bandreaktor) oder in Apparaten mit Rotoren ausgeführt, wobei die Reaktionsmasse jeweils bis zur Gelphase gemischt und geknetet wird.
Aus EP-B 223 063 ist ein Verfahren zur kontinuierlichen Herstellung von vernetzten feinteiligen gelförmigen Polymerisaten bekannt, bei dem man vorneutralisierte Acrylsäure mit Comonomeren in wässriger Lösung in einem kontinuierlich arbeitenden einwelligen, zylindrischen Kneter bei 45 - 80 °C zur Reaktion bringt und am Ende Gelklümpchen mit 30 bis 70 % Restfeuchte austrägt. Nachteilig bei diesem Verfahren ist die weitere Verarbeitung des zähen Gels mit aufwendiger Trocknung, Zerkleinerung und Siebung auf die gewünschte Korngröße. Staub und nicht erwünschte Feinanteile müssen verworfen oder anderweitig eingesetzt oder aufgearbeitet werden. Ferner werden bei höherem Feststoffgehalt durch die intensive Scherbeanspruchung des Gels die Molekülketten zerstört und damit die gewünschten Eigenschaften des Produkts nachteilig beeinflußt.
Die DE-A 3519013 beschreibt die Herstellung pulverförmiger, wasserlöslicher Polymerisate durch Polymerisation wasserlöslicher, ethylenisch ungesättigter Monomerer in einem Pulverbett, wobei wasserunlösliche Polymerisationsinitiatoren in Form flüssiger, organischer Peroxide eingesetzt werden. Durch Verdampfen von Wasser wird dabei die Polymerisationswärme unter Beibehaltung des umgewälzten Pulverbettes abgeführt.
Die DE-A 3842184 beschreibt ein Verfahren, bei dem wasserlösliche monoethylenisch ungesättigte Monomere in einem Pulverbett zu pulverförmigen hydrophilen Polymerisaten polymerisiert werden. Die Teilchengröße wird dabei dadurch gesteuert, daß die Lösung der Monomeren zusammen mit einer inerten Hilfsflüssigkeit mit Hilfe einer ins Bett getauchten Mehrstoffdüse verdüst wird. Der inerte Zerstäubungsgasstrom regelt durch seine Mengenvariation die Teilchengröße der Polymerisate.
DE-A 3842185 beschreibt ein Verfahren zur Herstellung pulverförmiger Polymerisate aus Acryl- und Methacrylsäure in einem Pulverbett. Die Monomeren werden in einer Mischung aus Wasser und Alkoholen mit Polymerisationinitiatoren und Regler unter Aufrechterhaltung des Pulverzustandes zugegeben, die Reaktionsmasse mechanisch umgewälzt und durch Abdestillieren der Lösemittel die Reaktionswärme abgeführt. Als Regler wird bei diesem Verfahren Thiocarbonsäure und/oder Mercaptoalkohol und Propionsäure und/oder Ameisensäure in einem definierten Mengenbereich eingesetzt.
EP-A 113048 beschreibt ebenfalls ein Verfahren zur Herstellung von pulverförmigen Polymerisaten auf der Basis wasserlöslicher ethylenisch ungesättigter Monomerer in einem bewegten Pulverbett, wobei als Vorrichtung Kessel, Rührautoklav oder Strömungsrohr in Betracht kommen. Die Monomeren werden dabei in Mischungen mit Wasser oder Wasser/Isopropanol unter dessen Aufrechterhaltung ins Pulverbett gegeben. Die Polymerisationswärme wird durch Abdestillieren der Lösungsmittel abgeführt. 40 - 95 % der Säuregruppen der Monomeren sind dabei neutralisiert und die Polymerisation läuft in Gegenwart von Thiocarbonsäure und weiteren Stoffen als Regler ab.

Die Verfahren gemäß DE-A 3519013, DE-A 3842185 und EP-A 113048 betreffen keine wasserquellbaren Polymerisate und haben somit keine Relevanz bezüglich vorliegender Erfindung.

Das Verfahren gemäß EP-B 223 063 hat den Nachteil, daß der Prozess über die Gelphase abläuft und höhere Feststoffanteile bei der weiteren Gelverarbeitung zu Schwierigkeiten durch zu hohe Energieaufnahme des Apparates und zu einer Verminderung der Produkteigenschaften führen. Ferner müssen die Gelpartikel getrocknet, gemahlen und gesiebt werden. Das unerwünschte Feinkorn muß recycliert oder verworfen werden, was aus ökonomischer und ökologischer Sicht unerwünscht ist.

Die DE-A 3842184 umfaßt auch die Herstellung vernetzter, Acrylsäure enthaltender Polymerisate. Allerdings wird hier kein Wirbelschichtpolymerisationsverfahren beschrieben, sondern ein Verfahren der Festbettpolymerisation, d. h. die Reaktionslösung wird auf ein mechanisch bewegtes bzw. gerührtes Festbett aus polymerisierten Partikeln gesprüht. Dabei muß ein inertes Verdünnungsmittel zugegeben werden, durch dessen Verdampfung die Polymerisationswärme abgeführt werden kann. Je nach Verdampfungstemperatur des inerten Lösungsmittels stellt sich eine Temperatur des Festbettes ein, d. h. daß die Polymerisationstemperatur auf Werte steigt, die für das Erzielen bestimmter Produkteigenschaften prohibitiv ist.
Als Vorrichtungen für dieses Verfahren werden Kessel, Rührautoklaven Kombinationen von Rührkessel und Strömungsrohr oder Rührkesselkaskaden genannt, also Vorrichtungen, die ein mechanisch bewegtes Pulverbett erzeugen. Im Gegensatz dazu wird in einer Wirbelschicht die Wärme vom im Fluidisierungsgas dispergierten, polymerisierten Partikel an das Gas leicht und entsprechend der Temperatur und Menge des zugeführten Fluidisierungsgases abgeführt. Dabei kann über die Eingangsparameter die Bettemperatur in dem für die Polymerisation und der sich daraus ergebenden Eigenschaften vorteilhafteren Bereich gehalten werden. Darüber hinaus wird durch die Dispergierung der Partikel deren Verkleben untereinander weit besser begegnet als im dicht mit Partikeln gepackten Pulverbett.

Aufgabe vorliegender Erfindung ist es, ein Verfahren zur Herstellung hydrophiler, hochquellfähiger Hydrogele bereitzustellen, das die oben genannten Nachteile nicht aufweist und bei dem möglichst kein Staub und keine Feinanteile anfallen.

Die vorliegende Erfindung betrifft deshalb ein Verfahren zur Herstellung hydrophiler, hochquellfähiger Hydrogele durch Polymerisation hydrophiler (Co)monomerer dadurch gekennzeichnet, daß die Polymerisation in einem Wirbelbettapparat ausgeführt wird.

Die vorliegende Erfindung betrifft bevorzugt ein Verfahren zur Herstellung hydrophiler, hochquellfähiger Hydrogele durch Polymerisation hydrophiler (Co)monomerer in Gegenwart von Wasser, wäßriger Lauge und Vernetzer, dadurch gekennzeichnet, daß die Polymerisation in einem Wirbelbettapparat ausgeführt wird, wobei
- (Co)monomere, Wasser, wäßrige Lauge und Vernetzer durch mit Druckgas beaufschlagte von unten nach oben sprühende Mehrstoffdüsen in eine Wirbelschicht gesprüht werden,
- die Mehrstoffdüsen in die Wirbelschicht getaucht sind,
- die Wirbelschicht durch einen heißen Inertgasstrom erzeugt wird, der durch einen Anströmboden und darüber befindliches Granulat von unten nach oben geleitet wird,
- durch den heißen Inertgasstrom die in der Wirbelschicht befindlichen Teilchen erwärmt werden und dadurch die Reaktion der auf diese gesprühten (Co)monomeren und Vernetzer ausgelöst und kontrolliert wird und
- die Trocknung dieser Teilchen unter Nutzung der Polymerisations- und/oder Neutralisationswärme im Wirbelbett stattfindet.

Das erfindungsgemäße Verfahren kann sowohl diskontinuierlich als auch kontinuierlich betrieben werden.

Gewünschtenfalls können zur Auslösung der Polymerisationsreaktion zusätzlich ein oder mehrere Polymerisationsinitiatoren eingesetzt werden.

Die Comonomeren können unmittelbar vor der Düseneinleitung mit der wässrigen Lauge, insbesondere Natron- oder Kalilauge in 30 - 50 %iger Konzentration, in einem statischen oder dynamischen Mischer, z. B. Supraton vorgemischt werden. Es ist aber auch möglich, die beiden Komponenten getrennt bis zur Düsenspitze zu führen, so daß die Neutralisation erst nach der Düse erfolgt.

Falls ein Initiator eingesetzt wird, so kann dieser dem Druckgas bei der Düseneinleitung mit einer Dosierpumpe, die einen höheren Druck als den Inertgasdruck erzeugt, eingespeist werden.

Der Feststoffgehalt in der Reaktionsmischung kann zwischen 30 und 90 Gew.% liegen, vorteilhaft zwischen 50 und 70 Gew.%. Besonders günstig sind Feststoffgehalte bei wässrigen Reaktionsmischungen, bei denen freiwerdende Neutralisations- und Polymerisationswärme den Wärmebedarf für die fühlbare Wärme und die Verdampfungsenthalpie des Wassers deckt. Beispielsweise liegt bei der Polymerisation der Acrylsäure dieser Bereich bei 60 - 65 Gew.% Feststoffgehalt.

Der Grad der Neutralisation hängt von dem gewünschten Anwendungsprofil des herzustellenden Granulates ab. Übliche pH-Werte des Granulates liegen bei 5,5 - 6,5.

Das erfindungsgemäße Verfahren kann bei Temperaturen im Wirbelbett von 60 bis 120 °C, vorzugsweise zwischen 80 und 105 °C ablaufen. Die Eintrittstemperatur des Wirbelgases in das Bett wird im wesentlichen durch den Feststoffgehalt und die Neutralisations- und Polymerisationswärme bestimmt und kann bis zu 30 °C unter und bis 80 °C über der Wirbelbettemperatur liegen.

Durch das Aufsprühen der Reaktionslösung auf vorhandene Partikel mit definierter Temperatur wird die in sehr dünner Schicht aufgebrachte Lösung sehr schnell umgesetzt und es bilden sich Granulate mit einem zwiebelförmigen Schalenaufbau.

Der Vorteil bei diesem Vorgehen liegt darin, daß die klebrige und sehr zähelastische Gelphase, die bei der Verarbeitung besonders bei Gelen mit höherer Feststoffkonzentration enorme Schwierigkeiten bringt, hierbei vermieden wird, da bei der Reaktion auch gleichzeitig die Trocknung in der dünnen Schicht erfolgt. Die Reaktion in der Wirbelschicht, d. h. in einer Gasströmung, in der die Teilchen dispergiert sind, vermeidet es außerdem, daß die Teilchen an den Wänden festkleben können. Durch die Bildung eines zwiebelförmigen Schalenaufbaus während der Reaktion besteht darüber hinaus die Möglichkeit, durch Variation der (Co)monomeren im zeitlichen Wechsel, unterschiedliche Eigenschaften im Granulat zu erzeugen.

Der Inertgasstrom zur Erzeugung der Wirbelschicht wird vor Eintritt in den Apparat auf die erforderliche Temperatur erhitzt und mit einer Durchsatzmenge von 2.000 bis 8.000 m³/m²h, vorzugsweise 5.000 bis 7.000 m³/m²h eingestellt. Der das Wirbelbett verlassende, mit Wasserdampf beladene Inertgasstrom wird in einem Zyklon oder druckgasabgereinigten Filter von mitgerissenen Feinanteilen befreit, die wiederum ins Wirbelbett zurückfallen.

Das erfindungsgemäße Verfahren läßt sich bei Monokomponentgranulaten besonders gut in kontinuierlicher Fahrweise einsetzen. Dabei können Forderungen nach definierten Kornverteilungen der Granulate in einfacher Weise erfüllt werden. Nach dem erfindungsgemäßen Verfahren ist die kontinuerliche Fahrweise bevorzugt dadurch gekennzeichnet, daß
a) eine Lösung der Reaktionskomponenten durch mit Druckgas beaufschlagte, von unten nach oben sprühende Mehrstoffdüsen in eine Wirbelschicht gesprüht wird, wobei ein gegebenenfalls eingesetzter Initiator getrennt bis zur Düsenspitze geführt und im Zerstäubungskegel mit der Lösung vermischt wird, wobei diese Wirbelschicht durch einen heißen Inertgasstrom erzeugt wird, der durch einen Anströmboden und darüber befindliches Granulat von unten nach oben geleitet wird, und wobei die Mehrstoffdüsen in die Wirbelschicht getaucht sind und
b) durch die Temperatur des eintretenden Inertgasstromes die Reaktion der auf die Oberfläche der Teilchen gesprühten Reaktionskomponenten im Wirbelbett kontrolliert erfolgt und dabei die Trocknung dieser Teilchen unter Nutzung der Polymerisations- und/oder Neutralisationswärme im Wirbelbett stattfindet und
c) kontinuierlich ein Granulatstrom aus dem Wirbelbett abgezogen und über ein Sieb geführt wird, das Überkorn auf einer Mühle zerkleinert und zusammen mit dem auf dem Sieb abgetrennten Unterkorn in die Wirbelschicht zurückgeführt wird, wobei diesem zurückzuführenden Material der Anteil an Gutkorn zugegeben wird, der die Menge an in Form der wässrigen Reaktionsmischung dem Wirbelbettapparat zugeführten Feststoff übersteigt und
d) der Feststoff über ein mittig oder außermittig am Boden angeordnetes Rohr entnommen wird, an dessen Ende eine Wirbelschicht durch einen kleinen von unten nach oben über ein Anströmblech geführten Inertgasstrom erzeugt wird, so daß im Auslaufrohr sich gleiche Verhältnisse wie in dem Wirbelbettapparat einstellen und eine gleichmäßige Entnahme über eine seitlich des Anströmbodens angebrachte Zellenradschleuse gewährleistet wird.

Der mit Wasserdampf beladene Inertgasstrom, verläßt über einen Zyklon oder vorteilhafter über ein druckgasabgereinigtes Filter den Wirbelbettapparat. Dieser Gasstrom kann rezirkuliert und wieder in die Wirbelschicht geführt werden. In diesem Fall wird der gesamte Strom über einen neutral oder alkalisch betriebenen Wäscherkondensator mit anschließendem Aerosolabscheider geführt. Aus diesem gereinigten Inertgasstrom werden die für den Austrag und die Druckgaskomprimierung erforderlichen Mengen entnommen und der Rest auf Eintrittstemperatur beheizt in das Wirbelbett eingeleitet.

Bei strengen Forderungen an die Kornklassen des herzustellenden Granulates erfolgt die Siebung mit mehreren Decks im Sieb, die den gewünschten Kornklassen entsprechende Maschenweiten der Siebbespannung aufweisen. Vorteilhafterweise ist dabei das obere Deck für die Obergrenze und das untere Deck für die Untergrenze der Kornverteilung bestimmend. Die Maschenweite der mittleren Decks wird dann so gewählt, daß eine mengenabgestimmte Ausschleusung von jeder

Deckfraktion eine spezifizierte Kornklasse im Gutkorn erzielt. Dabei wird mit Dosierschnecken der jeweilige Gewichtsanteil aus dem die Decks verlassenden Granulatstromes entnommen und die Einzelströme zum Produktstrom zusammengeführt. Der abgesiebte Überschuß an Gutkornklassen wird zusammen- und in den Wirbelbettapparat zurückgeführt.
Das Verhältnis von rezirkulierter Stoffmenge zu entnommener Gutkornmenge liegt vorzugsweise zwischen 1 und 10.
Die nach dem erfindungsgemäßen Verfahren hergestellten Granulate haben in der Regel Korngrößen von 100 µm bis 2 mm, vorzugsweise 200 bis 800 µm, besonders bevorzugt zwischen 300 und 600 µm.

Nach dem erfindungsgemäßen Verfahren können im diskoninuierlich ablaufenden Betrieb auch Bi- oder Mehrkomponentengranulate hergestellt werden, die dadurch gekennzeichnet sind, daß
a) im Wirbelbett Material, z. B. polymerisiertes Granulat, Naturstoffe, z. B. Stärkegranulat oder Inertstoffe vorgelegt wird und
b) unterschiedliche Reaktionsmischungen zeitlich hintereinander oder im zeitlichen Wechsel aufgesprüht und polymerisiert werden

Nach dem erfindungsgemäßen Verfahren gelingt es durch sich an die Polymerisationsreaktion anschließendes Aufsprühen von Modifizierungsmitteln im Wirbelbett, die Oberflächeneigenschaften der hydrophilen Hydrogele zu verändern, beispielsweise zu hydrophobieren oder zu hydrophilieren oder Permeabilität, Quellverhalten oder Saugfähigkeit usw. zu verändern.

Die Modifizierungsmittel sind in der Regel in Wasser oder einem Lösungsmittel gelöst oder emulgiert. Dabei wird durch die Temperatur des ins Wirbelbett eintretenden Inertgasstroms die Temperatur im Wirbelbett so gesteuert, daß die für Auslösung und Ablauf der Reaktion und/oder Trocknung des Modifizierungsmittel notwendigen Temperaturbedingungen eingehalten werden.

Das erfindungsgemäße Verfahren erfordert sowohl bei der diskontinuierlichen, als auch bei der kontinuierlichen Fahrweise, vor dem Einsprühen der Reaktionsmischung ein Granulat vorzulegen. Die Kornverteilung dieses Materials wird dem Anwendungsfall entsprechend ausgewählt. In der Regel legt man bei Monokomponentengranulaten ein Material vor, dessen Zusammensetzung dem Produkt gleicht, das hergestellt werden soll.

Für das erfindungsgemäße Verfahren besonders geeignete Monomere sind beispielsweise Acrylsäure, Methacrylsäure, Acrylamidopropansulfonsäure, Vinylphosphonsäure, Vinylsulfonsäure, Styrolsulfonsäure, Crotonsäure, Maleinsäure, Maleinsäurehalbester, Maleinsäureanhydrid, Maleinsäurehalbamide, Acrylamide, Methacrylamide, Vinylpyrrolidone, Vinylamide wie N-Vinyl-N-methylacetamid, N-Vinylformamid, N-Vinylcaprolactam, Hydroxyalkylester der Acryl- oder Methacrylsäure, Vinylpyridine, N.N-Dimethyl-Diallylammoniumchlorid, Aminoalkylester sowie Aminoalkylamide der Acryl- und Methacrylsäure.

Bevorzugt erfolgt der Einsatz dieser Monomeren in Form wässriger Lösungen. Soweit es sich um Säuren handelt können sie in Form der freien Säuren oder in Form der Alkali-, Ammonium- oder Aminsalze sowie deren Mischungen eingesetzt werden. Es können nach dem erfindungsgemäßen Verfahren auch Mischungen der genannten Monomeren eingesetzt werden. Besonders bevorzugte Monomere sind Acrylsäure und Methacrylsäure.

Weitere Comonomerenkomponenten, welche bis zu 30 Gew.% der Gesamtmonomermenge eingesetzt werden können, sind beispielsweise (C₁-C₂₂)-Ester der Acryl-, Methacryl- oder Maleinsäure sowie Polyoxalkylenester dieser Säure, Vinylester wie Vinylacetat oder Versaticsäure sowie Polyoxalkylenester dieser Säuren, Vinylester wie Vinylacetat oder Versaticsäurevinylester, Styrol, Vinyltoluol und Acrylnitril.

Als Vernetzer sind nach dem erfindungsgemäßen Verfahren Verbindungen einsetzbar, welche mehr als eine olefinisch ungesättigte Gruppe im Molekül enthalten, z. B. Acryl- und Methacrylsäureester mehrwertige Alkohole, wie z. B. Trimethylolpropantriacrylat, Butandioldimethacrylat oder Allyläther mehrwertiger Alkohole, z. B. Neopentylglykoldiallyläther, Tetraallyloxäthan, Allylamine wie Triallylamin oder Tetraallylammoniumchlorid, sowie Divinylbenzol, Divinylsulfon, Adipinsäuredivinylester und N-Methylenbisacrylamid.

Als Polymerisationsinitiatoren nach dem erfindungsgemäßen Verfahren können gegebenenfalls die üblichen Perverbindungen, wie Dibenzoylperoxyd, tert. Butylhydroperoxid, Cumolhydroperoxid, Kalium-, Natrium- und Ammoniumperoxodisulfat, sowie Azoinitiatoren wie Azoisobutyronitril eingesetzt werden. Vorzugsweise werden Redoxsysteme, wie Z. B. Wasserstoffperoxid-Ascorbinsäure, Peroxodisulfat-Na-pyrosulfit, Aldehydsulfoxylate-Peroxyde eingesetzt.

Für das erfindungsgemäße Verfahren geeignete Modifizierungskomponenten sind flüssige Stoffe, wie z. B. Polyalkylenoxide, insbesondere Polyethylenglykole, Polypropylenglykole, Polyglykole mit einem Molekulargewicht bis ca. 600, Paraffine, Polyamine, wie z. B. Ethylendiamin, Diethylentriamin, Polyethylenimin, Polyglycidylverbindungen, wie z. B. Ethylenglykoldiglycidylether, Propylenglykoldiglycidylether, Polyethylenglykoldiglycidylether, Glyzerinpolyglycidylether, flüssige mehrwertige Alkohole, wie z. B. Glyzerin, Pentaerythrit, Trimethylolpropan, Neopentylalkohol, Sorbitol, Polyvinylalkohol, sowie Lösungen von Poly(Meth)-acrylaten, Polyamidoaminen, Polyvinylacetat und Copolymeren.
Besonders bevorzugt sind Diglycidylverbindungen, Polyglycidylverbindungen und Polyamine, beispielsweise Ethylenglykoldiglycidylether, Polyethylenglykoldiglycidylether, Diethylentriamin und Polyethylenimin.
Modifizierungskomponenten werden bei dem erfindungsgemäßen Verfahren, soweit sie keine Gruppierungen im Molekül eingebaut enthalten, welche mit der Oberfläche der Hydrogelpartikel chemisch reagieren, adsorptiv an der Oberfläche gebunden. Modifizierungskomponenten, welche Gruppierungen enthalten, die in der Lage sind, mit der Oberfläche der Hydrogelpartikel chemisch zu reagieren, können kovalente, ionische oder Komplexbindungen eingehen, wie z. B. Polyglycidylverbindungen, Polyanionen, Polykationen oder mehrwertige Metallkationen.

## Patentansprüche

1. Verfahren zur Herstellung hydrophiler, hochquellfähiger Hydrogele durch Polymerisation hydrophiler (Co)monomerer, dadurch gekennzeichnet, daß die Polymerisation in einem Wirbelbettapparat ausgeführt wird.

2. Verfahren zur Herstellung hydrophiler, hochquellfähiger Hydrogele durch Polymerisation hydrophiler (Co)monomerer in Gegenwart von Wasser wäßriger Lauge und Vernetzer, dadurch gekennzeichnet, daß die Polymerisation in einem Wirbelbettapparat ausgeführt wird, wobei
- (Co)monomere, Wasser, wäßrige Lauge und Vernetzer durch mit Druckgas beaufschlagte von unten nach oben sprühende Mehrstoffdüsen in eine Wirbelschicht gesprüht werden,
- die Mehrstoffdüsen in die Wirbelschicht getaucht sind,
- die Wirbelschicht durch einen heißen Inertgasstrom erzeugt wird, der durch einen Anströmboden und darüber befindliches Granulat von unten nach oben geleitet wird,
- durch den heißen Inertgasstrom die in der Wirbelschicht befindlichen Teilchen erwärmt werden und dadurch die Reaktion der auf diese gesprühten (Co)monomeren und Vernetzer ausgelöst und kontrolliert wird und
- die Trocknung dieser Teilchen unter Nutzung der Polymerisations- und/oder Neutralisationswärme im Wirbelbett stattfindet.

3. Verfahren gemäß Anspruch 1 und/oder 2, dadurch gekennzeichnet, daß zur Auslösung der Polymerisationsreaktion ein oder mehrere Polymerisationsinitiatoren eingesetzt werden.

4. Verfahren gemäß einem oder mehreren der Ansprüche 1 bis 3, dadurch gekennzeichnet, daß der Feststoffgehalt der Reaktionsmischung zwischen 30 und 90 Gew.% liegt.

5. Verfahren gemäß einem oder mehreren der Ansprüche 1 bis 4, dadurch gekennzeichnet, daß der Inertgasstrom zur Erzeugung der Wirbelschicht vor Eintritt in den Apparat auf die erforderliche Temperatur erhitzt und mit einer Durchsatz-menge von 2.000 bis 8.000 m³/m²h eingestellt wird.

6. Verfahren gemäß einem oder mehreren der Ansprüche 1 bis 5, dadurch gekennzeichnet, daß kontinuierlich gefahren wird, wobei
a) eine Lösung der Reaktionskomponenten durch mit Druckgas beaufschlagte, von unten nach oben sprühende Mehrstoffdüsen in eine Wirbelschicht gesprüht wird, wobei ein gegebenenfalls eingesetzter Initiator getrennt bis zur Düsenspitze geführt und im Zerstäubungskegel mit der Lösung vermischt wird, wobei diese Wirbelschicht durch einen heißen Inertgasstrom erzeugt wird, der durch einen Anströmboden und darüber befindliches Granulat von unten nach oben geleitet wird, und wobei die Mehrstoffdüsen in die Wirbelschicht getaucht sind und
b) durch die Temperatur des eintretenden Inertgasstromes die Reaktion der auf die Oberfläche der Teilchen gesprühten Reaktionskomponenten im Wirbelbett kontrolliert erfolgt und dabei die Trocknung dieser Teilchen unter Nutzung der Polymerisations- und/oder Neutralisationswärme im Wirbelbett stattfindet und
c) kontinuierlich ein Granulatstrom aus dem Wirbelbett abgezogen und über ein Sieb geführt wird, das Überkorn auf einer Mühle zerkleinert und zusammen mit dem auf dem Sieb abgetrennten Unterkorn in die Wirbelschicht zurückgeführt wird, wobei diesem zurückzuführenden Material der Anteil an Gutkorn zugegeben wird, der die Menge an in Form der wässrigen Reaktionsmischung dem Wirbelbettapparat zugeführten Feststoff übersteigt und
d) der Feststoff über ein mittig oder außermittig am Boden angeordnetes Rohr entnommen wird, an dessen Ende eine Wirbelschicht durch einen kleinen von unten nach oben über ein Anströmblech geführten Inertgasstrom erzeugt wird, so daß im Auslaufrohr sich gleiche Verhältnisse wie in dem Wirbelbettapparat einstellen und eine gleichmäßige Entnahme über eine seitlich des Anströmbodens angebrachte Zellenradschleuse gewährleistet wird.

7. Verfahren gemäß einem oder mehreren der Ansprüche 1 bis 6, dadurch gekennzeichnet, daß die hergestellten Granulate Korngrößen von 100 µm bis 2 mm aufweisen.

8. Verfahren gemäß einem oder mehreren der Ansprüche 1 bis 7, dadurch gekennzeichnet, daß durch sich an die Polymerisationsreaktion anschließendes Aufsprühen von Modifizierungsmitteln im Wirbelbett die Oberflächeneigenschaften der hydrophilen Hydrogele verändert werden.

9. Verfahren gemäß einem oder mehreren der Ansprüche 1 bis 8, dadurch gekennzeichnet, daß als (Co)monomere Acrylsäure, Methacrylsäure, Acrylamidopropansulfonsäure, Vinylphosphonsäure, Vinylsulfonsäure, Styrolsulfonsäure, Crotonsäure, Maleinsäure, Maleinsäurehalbester, Maleinsäureanhydrid, Maleinsäurehalbamide, Acrylamide, Methacrylamide, Vinylpyrrolidone, Vinylamide wie N-Vinyl-N-methylacetamid, N-Vinylformamid, N-Vinylcaprolactam, Hydroxyalkylester der Acryl- oder Methacrylsäure, Vinylpyridine, N.N-Dimethyl-Diallylammoniumchlorid, Aminoalkylester oder Aminoalkylamide der Acryl- und Methacrylsäure eingesetzt werden.

10. Verfahren gemäß einem oder mehreren der Ansprüche 1 bis 9, dadurch gekennzeichnet, daß als Modifizierungsmittel flüssige Stoffe, wie z. B. Polyalkylenoxide, insbesondere Polyethylenglykole, Polypropylenglykole, Polyglykole mit einem Molekulargewicht bis ca. 600, Paraffine, Polyamine, wie z. B. Ethylendiamin, Diethylentriamin, Polyethylenimin, Polyglycidylverbindungen, wie z. B. Ethylenglykoldiglycidylether, Propylenglykoldiglycidylether, Polyethylenglykoldiglycidylether, Glyzerinpolyglycidylether, flüssige mehrwertige Alkohole, wie z. B. Glyzerin, Pentaerythrit, Trimethylolpropan, Neopentylalkohol, Sorbitol, Polyvinylalkohol, sowie Lösungen von Poly(Meth)-acrylaten, Polyamidoaminen, Polyvinylacetat oder Copolymeren eingesetzt werden.
